# EUROPEAN PATENT APPLICATION

(11) **EP 4 454 543 A1**
(43) Date of publication of application: **30.10.2024**
(21) Application number: 22911331.1
(22) Date of filing: 22.12.2022
(51) Int. Cl.: A61B 3/135

(54) **MEDICAL EXAMINATION DEVICE**

(30) Priority: 23.12.2021 JP 2021208955
(71) Applicant: KOWA COMPANY, LTD., Naka-ku Nagoya-shi Aichi 460-8625 (JP)
(72) Inventor: KAKUUCHI, Atsushi, Chofu-shi Tokyo 182-0021 (JP)
(74) Representative: Mathys & Squire
(86) International application number: PCT/JP2022/047308
(87) International publication number: WO 2023/120629

(57) **Abstract**

To provide a medical examination device having a plurality of irradiation lights that can be independently controlled while achieving downsizing, the medical examination device includes: an observation optical system that observes a subject; an illumination optical system including a slit light generation unit that generates slit light, a first light generation unit that generates first light different from the slit light, and an exterior in which a slit light irradiation port that emits the slit light and a first light irradiation port that emits the first light are formed at different positions; and a control unit that controls the illumination optical system. The first light generation unit includes a first light source disposed at a position where the first light can be emitted from the first light irradiation port.

## Description

### Technical Field

The present invention relates to a medical examination device for examining a subject.

### Background Art

Conventionally, in the medical field, a hand-held examination device for examining a subject such as a human or an animal has been used. Patent Literature 1 discloses an examination device capable of examining an anterior ocular segment by irradiating an eye, which is a subject, with slit light while being held by an operator (user) with one hand.

### Citation List

### Patent Literature

Patent Literature 1: JP 4136620 B2

### Summary of Invention

### Technical Problem

Here, in the conventional ophthalmic examination device as in Patent Literature 1, there is a configuration in which light emitted from a light source is converted into slit light by passing through a slit, and the slit light is emitted to an examinee's eye, and a configuration in which irradiation with the slit light and irradiation with illumination light illuminating a wide range of the examinee's eye can be switched by enabling insertion and removal of a slit at a subsequent stage of the light source. Since a hand-held examination device is desired to be small, it can be said that it is desirable to reduce the size by allowing one light source to play two roles, but there is a problem that both cannot be used in combination in a case where it is desired to further irradiate the examinee's eye with illumination light (background illumination) in a state where the examinee's eye is irradiated with slit light, for example, in a case where it is desired to photograph the examinee's eye with a camera in a state where the subject's eye is irradiated with slit light. For this reason, there has been a demand for an examination device capable of independently controlling and irradiating a plurality of irradiation lights.

On the other hand, since the small size of a hand-held examination device has an aspect that leads to good usability, there is a circumstance that it is desired to avoid an increase in size of the examination device even if a plurality of irradiation lights can be independently controlled and emitted.

The present invention has been made in view of the above problems, and an object of the present invention is to provide a medical examination device capable of independently controlling and irradiating a plurality of irradiation lights while realizing miniaturization.

### Solution to Problem

A medical examination device according to the present invention includes: an observation optical system that observes a subject; an illumination optical system including a slit light generation unit that generates slit light, a first light generation unit that generates first light different from the slit light, and an exterior in which a slit light irradiation port that emits the slit light and a first light irradiation port that emits the first light are formed at different positions; and a control unit that controls the illumination optical system. The first light generation unit includes a first light source disposed at a position where the first light can be emitted from the first light irradiation port.

In addition, in the medical examination device according to the present invention, the illumination optical system may include an irradiation tube that allows the slit light emitted from the slit light generation unit to pass therethrough, reflects the slit light at an end portion, and irradiates the subject with the slit light, and a substrate on which the first light source is mounted is disposed as the first light generation unit between a passing region of the slit light inside the irradiation tube and an outer wall (exterior) of the irradiation tube so as not to enter the passing region of the slit light.

In addition, in the medical examination device according to the present invention, the substrate may be extended in a longitudinal direction of the irradiation tube to secure an area necessary for a circuit configuration.

In addition, in the medical examination device according to the present invention, the control unit may independently control irradiation with the slit light and irradiation with the first light.

In addition, in the medical examination device according to the present invention, the first light generation unit may include a first diffusion plate and emits the first light from the first light irradiation port via the first diffusion plate.

In addition, in the medical examination device according to the present invention, the first light irradiation port may have an opening that limits an illumination area of the first light such that the center of a visual field of the observation optical system and the center of the illumination area of the first light substantially coincide with each other.

In addition, in the medical examination device according to the present invention, the illumination optical system may include a second light generation unit that generates a second light different from the slit light and the first light, and the exterior of the illumination optical system has a second light irradiation port that emits the second light in the vicinity of the first light irradiation port.

In addition, the medical examination device according to the present invention may include a grip unit that can be held by a user with one hand. The grip unit includes a slit light button configured to instruct start/stop of irradiation with the slit light, a first light button configured to instruct start/stop of irradiation with the first light, and a second light button configured to instruct start/stop of irradiation with the second light, and the slit light button, the first light button, and the second light button are arranged to be operable by the user's holding hand in a state where the user holds the grip unit.

In addition, in the medical examination device according to the present invention, in a state where the user holds the grip unit, the slit light button and the second light button may be arranged to be operable by different fingers, and the first light button and the second light button are arranged so as to be operable by the same finger.

In addition, in the medical examination device according to the present invention, the second light generation unit may include a second diffusion plate and emit the second light from the second light irradiation port via the second diffusion plate.

In addition, in the medical examination device according to the present invention, the control unit may control to prohibit the irradiation with the second light during the irradiation with the slit light and to prohibit the irradiation with the slit light during the irradiation with the second light.

In addition, in the medical examination device according to the present invention, the control unit controls to exclusively emit the first light and the second light.

In addition, in the medical examination device according to the present invention, the second light irradiation port may have an opening that limits an illumination area of the second light such that the center of a visual field of the observation optical system and the center of the illumination area of the second light substantially coincide with each other.

### Advantageous Effects of Invention

Embodiments of the present application solve one or more deficiencies.

### Brief Description of Drawings

Fig. 1 is an external view illustrating an example of a configuration of a medical examination device 100 corresponding to at least one of embodiments of the present invention.
Fig. 2 is an external view illustrating an example of a configuration of the medical examination device 100 corresponding to at least one of the embodiments of the present invention.
Fig. 3 is an external view illustrating an example of a configuration of the medical examination device 100 corresponding to at least one of the embodiments of the present invention.
Fig. 4 is an external view when an operator holds the medical examination device 100 corresponding to at least one of the embodiments of the present invention with one hand.
Fig. 5 is a perspective view illustrating an example of a configuration of a portion of an irradiation unit 10 in the medical examination device 100 corresponding to at least one of the embodiments of the present invention.
Fig. 6 is a cross-sectional view illustrating an example of a configuration of a portion of the irradiation unit 10 in the medical examination device 100 corresponding to at least one of the embodiments of the present invention.
Fig. 7 is a cross-sectional view illustrating an example of a configuration of a portion of the irradiation unit 10 in the medical examination device 100 corresponding to at least one of the embodiments of the present invention.
Fig. 8 is a cross-sectional view illustrating an example of a configuration of a portion of the irradiation unit 10 in the medical examination device 100 corresponding to at least one of the embodiments of the present invention.
Fig. 9 is a cross-sectional view illustrating an example of a configuration of a portion of the irradiation unit 10 in the medical examination device 100 corresponding to at least one of the embodiments of the present invention.
Fig. 10 is an explanatory diagram illustrating an example of a relationship between an irradiation state of each irradiation light and availability of button operation in the medical examination device 100 corresponding to at least one of the embodiments of the present invention.

### Description of Embodiments

Hereinafter, an example of a medical examination device according to embodiments of the present invention will be described with reference to the drawings. The medical examination device according to the present example is a so-called slit lamp, and is a device for examining a cornea, a crystalline lens, or the like of an examinee's eye by irradiating an eye (hereinafter, the eye is referred to as an "examinee's eye") of a subject, which is a subject with slit light and observing scattered light generated by scattering in the examinee's eye. Hereinafter, a case where a medical examination device is a hand-held slit lamp will be described as an example.

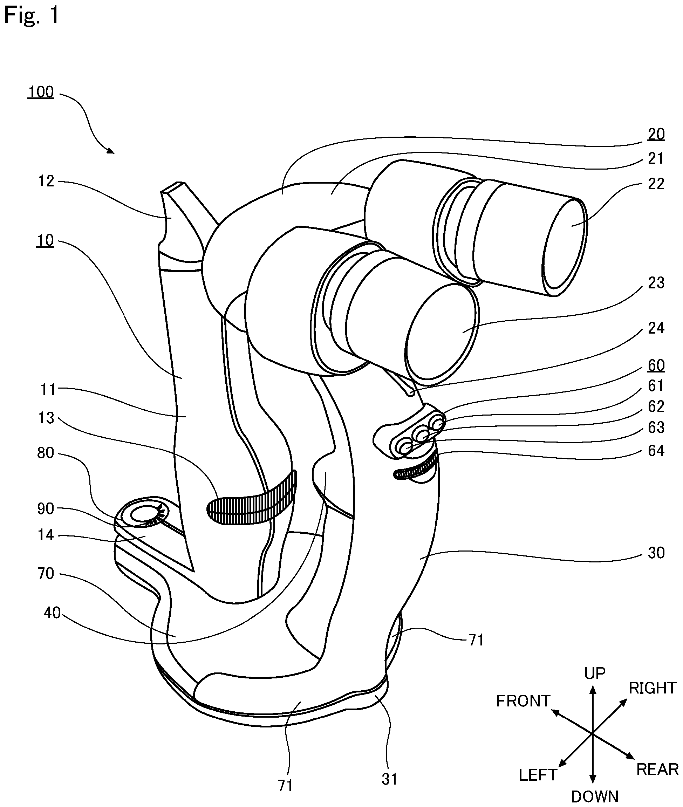

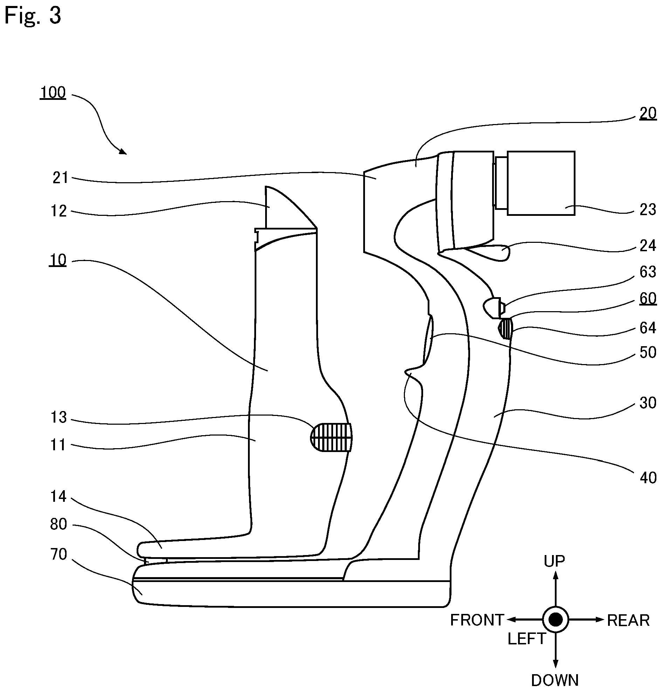

Figs. 1 to 3 are external views illustrating an example of a configuration of a medical examination device 100 corresponding to embodiments of the present invention. The medical examination device 100 is a device for examining the examinee's eye by operating in a state of being held with one hand. As illustrated in Figs. 1 to 3, the medical examination device 100 includes an irradiation unit 10, an observation unit 20, a grip unit 30, a finger hook unit 40, an index finger side operation unit 50, a thumb side operation unit 60, a base unit 70, an irradiation angle adjustment unit 80, and a scale unit 90. Furthermore, in the present example, there is a case where the irradiation unit 10 is expressed as an illumination optical system and the observation unit 20 is expressed as an observation optical system, and a description will be given on the assumption that a control unit that controls the illumination optical system is provided inside.

The irradiation unit (illumination optical system) 10 has a function of irradiating the examinee's eye with irradiation light. In the present example, the irradiation unit 10 may have a function of irradiating the examinee's eye with at least slit light as irradiation light, and may further have a function of irradiation with spot light. The irradiation unit 10 includes, for example, an irradiation tube 11, an irradiation port 12, a disk operation unit 13, a swing unit 14, and a separate light source installation unit 15. The irradiation tube 11 has a known configuration for irradiation with slit light, as described in Patent Literature 1 described above, arranged inside. In the present example, the irradiation tube 11 includes a light source, a condenser lens, a spot disk, a slit disk, and a light projecting lens therein. An example of the light source is an LED. In the irradiation tube 11, light emitted from the light source is condensed by the condenser lens, and then passes through the slit disk to generate slit light and spot light. The slit light and the spot light are incident on the irradiation port 12. The irradiation port 12 includes a light projecting prism disposed inside. The slit light and the spot light incident on the light projecting prism change directions and are emitted toward the examinee's eye outside the device. The disk operation unit 13 is a dial for an operator (user) to rotate the spot disk and the slit disk and select the length and the width of the slit light to be emitted and the diameter of the spot light. The swing unit 14 is formed of a plate-like member which is provided at one end portion and a lower portion of the irradiation tube 11, and has the other end portion positioned at a predetermined distance from the irradiation tube 11 and connected to the irradiation angle adjustment unit 80 to be described later. Note that a marker for indicating the rotation angle of the irradiation unit 10 is provided in a portion in contact with the outer periphery of the irradiation angle adjustment unit 80 in the swing unit 14.

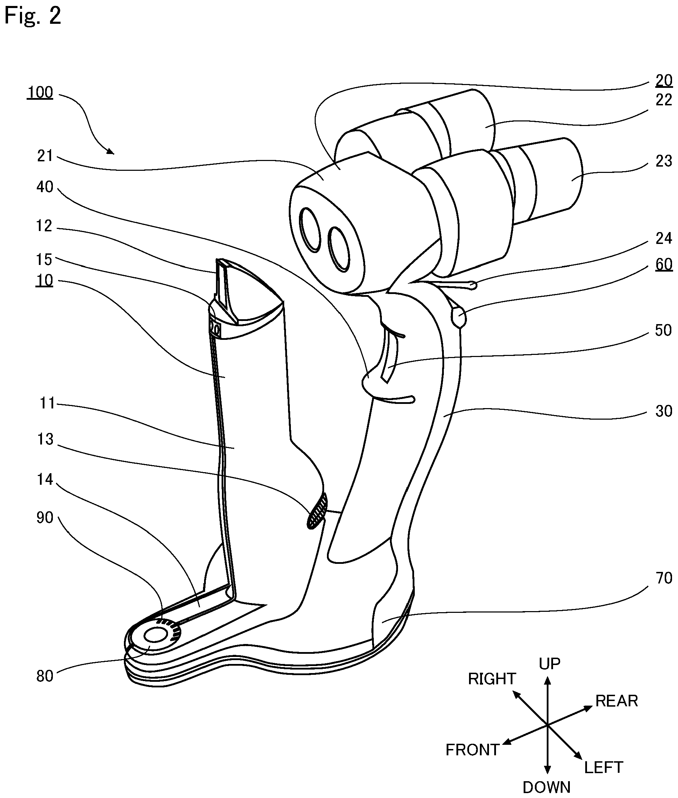

The separate light source installation unit 15 is a configuration for installing a light source different from the slit light, and is provided separately from the irradiation port 12. As the light source provided in the separate light source installation unit 15, for example, a background illumination light source (first light source) and a fluorescence observation illumination light source (second light source) can be considered. For example, a white LED is used as the background illumination light source, and for example, a blue LED is used as the fluorescence observation illumination light source. Note that the background illumination light source and the fluorescence observation illumination light source are examples of light sources provided in the separate light source installation unit 15, and thus are not limited thereto. Conventionally, light of background illumination or fluorescence observation illumination is emitted from the same irradiation port as slit light or spot light, but it is necessary to switch between the slit light or the spot light and light of background illumination or light of fluorescence observation illumination, and simultaneous irradiation cannot be performed. However, since the light source is directly installed in the separate light source installation unit 15 provided in the medical examination device 100, it is possible to emit light of background illumination or light of fluorescence observation illumination at the time of irradiation with slit light or spot light. As a result, for example, it is possible to use for photographing or the like for explaining the irradiation state of the slit light to the subject (for example, photographing using background illumination of the anterior ocular segment of the subject as the subject). In addition, since the light of the background illumination and the fluorescence observation illumination is generated in an optical path different from an optical path of the slit light and the spot light, an irradiation range is not limited by the size of a slit turret, and the light source can be installed so that the background illumination and the fluorescence observation illumination are illuminated in a wider range as compared with the case where the light is generated in the same optical path. In addition, as illustrated in Fig. 2, the separate light source installation unit 15 is provided so that the light emitted from the irradiation port 12 and the light emitted from the separate light source installation unit 15 always face substantially the same direction. For example, the separate light source installation unit 15 similarly rotates in accordance with the rotation of the irradiation port 12. With such a configuration, it is not necessary to separately adjust the direction of the background illumination and the fluorescence observation illumination when adjusting the irradiation direction of the slit light and the spot light, and as a result, it is possible to improve the ease of handling of the medical examination device 100.

The observation unit (observation optical system) 20 has a function of observing the examinee's eye with the irradiation light emitted from the irradiation unit 10. In the present example, the observation unit 20 includes an observation casing 21, a right-eye eyepiece 22, a left-eye eyepiece 23, and a zoom lever 24. The observation casing 21, the right-eye eyepiece 22, and the left-eye eyepiece 23 are internally configured by the known observation optical system described in Patent Literature 1 described above. The observation optical system is divided into an observation for the right eye and an observation for the left eye. The observation optical system for the right eye is configured inside the observation casing 21 and the right-eye eyepiece 22. In addition, the observation optical system for the left eye is configured inside the observation casing 21 and the left-eye eyepiece 23. For example, the observation optical system includes at least an objective lens, an eyepiece prism, a reticle lens, and an eyepiece lens. The slit light from the irradiation unit 10 is scattered by the examinee's eye and is incident on the objective lens as scattered light. The incident scattered light passes through the eyepiece prism, the reticle lens, and the eyepiece lens, and becomes light that can be observed by the operator. The zoom lever 24 is a lever that can move in the left-right direction to move the objective lens in the front-back direction and change the observation magnification of the examinee's eye.

The grip unit 30 is provided so as to be holdable by the operator so as to be sandwiched between the thumb of one hand and at least one of the four fingers on the index finger side (hereinafter, also referred to as "index finger side finger"). The shape of the grip unit 30 is not particularly limited as long as the operator can hold the grip unit, but a substantially cylindrical shape is preferable. In the present example, as illustrated in Figs. 1 to 3, the grip unit 30 is attached to the lower portion of the observation casing 21 and has a shape like a curved cylinder. Hereinafter, a state in which the central axis of the grip unit 30 is oriented in a substantially vertical direction (vertical direction in Figs. 1 to 3) is referred to as a "reference state". The direction of the central axis of the grip unit 30 illustrated in Figs. 1 to 3 is assumed to be a substantially vertical direction. Therefore, the state illustrated in Figs. 1 to 3 is the reference state.

The finger hook unit 40 is provided on the grip unit 30 so as to be hooked on any of the fingers located on the index finger side when the operator holds the grip unit 30 with one hand. Here, the index finger side refers to a side on which the index finger can come into contact when the grip unit 30 is held among the side surfaces of the grip unit 30. The finger located on the index finger side is at least one of an index finger, a middle finger, a ring finger, and a little finger. In addition, the finger hook unit 40 being hooked on the finger means that the finger is in a state of receiving a load of the finger hook unit 40. The shape of the finger hook unit 40 is not particularly limited as long as it can be hooked on any of the fingers located on the index finger side. In the example shown in Figs. 1 to 3, the shape of the finger hook unit 40 is a shape protruding in a flange shape from the side surface of the grip unit 30. By providing the finger hook unit 40 on the grip unit 30, the operator can hold the grip unit 30 by hooking the finger hook unit 40 on any of the fingers located on the index finger side. Therefore, it is possible for the operator to stably perform the operation for examination at the ideal position of the hand holding the grip unit 30 defined by the finger hook unit 40. An example of the shape of the finger hook unit will be described later.

The index finger side operation unit 50 is used for the operation for examination, and is provided at a position within a range where a finger assumed to be used for the operation in advance reaches, among the fingers on the index finger side whose position is determined by the finger hook unit 40 when the operator holds the grip unit 30 with one hand. The finger assumed to be used for the operation in advance is not particularly limited as long as it is any of the fingers on the index finger side, but a finger other than the finger on which the finger hook unit 40 is hooked is preferable. In the present example, the index finger is assumed as the finger used for the operation. In addition, the position within the range that the finger reaches refers to a position that the finger reaches by moving the finger. An example of the position within the range where the finger assumed to be used for the operation reaches in advance is the position of the grip unit 30 facing the finger assumed to be used for the operation in advance when the operator holds the grip unit 30 with one hand. The index finger side operation unit 50 is, for example, a button, a switch, or the like. In the present example, the index finger side operation unit 50 is a switch that, while being pressed, causes the light source disposed in the irradiation tube 11 of the irradiation unit 10 to emit light and the light is emitted from the irradiation port 12.

Further, the index finger side operation unit 50 may be provided at a position in a range where a finger for the operation reaches, assuming that the finger for the operation is a finger closer to the thumb than a finger receiving a load from the finger hook unit 40. The finger closer to the thumb than the finger receiving the load is, for example, the index finger when the finger receiving the load in the reference state is the middle finger. With such a configuration, it is possible to more stably perform the operation for examination with the finger having a relatively small influence of the load from the finger hook unit 40.

The thumb side operation unit 60 is used for the operation for examination of the examinee's eye, and is provided at a position within a range where the thumb, whose position is determined by the finger hook unit 40 when the operator holds the grip unit 30 with one hand, reaches. The thumb side operation unit 60 may be provided with a plurality of operation portions. The operation portions are provided with operation members such as buttons, various switches, and dials for performing operation input related to the examination of the examinee's eye. In the present example, operation buttons 61 to 63 and an operation dial 64 are provided as operation members at the operation portions of the thumb side operation unit 60. Various functions related to examination such as on/off of background illumination and fluorescence observation illumination are assigned to the operation buttons 61 to 63. Furthermore, the operation dial 64 is assigned a function of adjusting the amount of light of the slit light emitted from the irradiation port 12 of the irradiation unit 10, for example. An operation portion of the plurality of operation buttons included in the thumb side operation unit 60 is not particularly limited as long as the operation portion is positioned within a range where the thumb, whose position is determined by the finger hook unit 40 when the operator holds the grip unit 30 with one hand, reaches. Examples of the shape, size, arrangement, and the like of the plurality of operation members included in the thumb side operation unit 60 will be described later.

The base unit 70 is a plate-like member having one end portion attached to a lower portion of the grip unit 30. At the other end portion of the base unit 70, the irradiation angle adjustment unit 80 to be described later is provided.

The irradiation angle adjustment unit 80 is a member that adjusts the irradiation angle of irradiation light by rotating the irradiation unit 10 with respect to the rotation axis. In the present example, the irradiation angle adjustment unit 80 is fixedly attached to the other end portion of base unit 70. In the present example, the irradiation angle adjustment unit 80 is rotatably attached to a hole formed at a portion at a predetermined distance from the irradiation tube 11 in the swing unit 14, and the upper surface is exposed. That is, the base unit 70 and the swing unit 14 are connected via the irradiation angle adjustment unit 80, and the swing unit 14 is rotatable with respect to the base unit 70 based on the irradiation angle adjustment unit 80. Note that the irradiation angle adjustment unit 80 is configured by a bearing, for example.

The scale unit 90 is provided with a scale indicating a rotation angle on the upper surface of the rotation shaft. Specifically, the scale unit 90 is provided with a scale corresponding to the irradiation angle of the irradiation light emitted by the irradiation unit 10 in an arc shape on the upper surface of the rotation shaft. In the present example, the scale unit 90 is provided as a mark indicating a rotation angle of the swing unit 14 with respect to the irradiation angle adjustment unit 80 in an arc shape on the upper surface of the irradiation angle adjustment unit 80 fitted to the swing unit 14. The scale unit 90 will be described later in detail.

The example of the configuration and the appearance of the medical examination device 100 has been described above. Note that the medical examination device 100 may be a device configured to perform load balancing such that the center of gravity of the entire device is located on the index finger side when the thumb side and the index finger side are divided into two by a reference plane that is a virtual plane parallel to the central axis and passes through the position of the finger hook unit 40. With such a load balance, even if the grip of the hand of the grip unit 30 is loosened, the medical examination device 100 is inclined from the thumb side to the index finger side with reference to the finger hook unit 40. Since the inclination occurs with reference to the finger hook unit 40, for example, a state in which the finger hook unit 40 is hooked on the finger is maintained in the reference state. As a result, even if the grip of the hand is loosened, the state of holding the grip unit 30 can be more easily maintained.

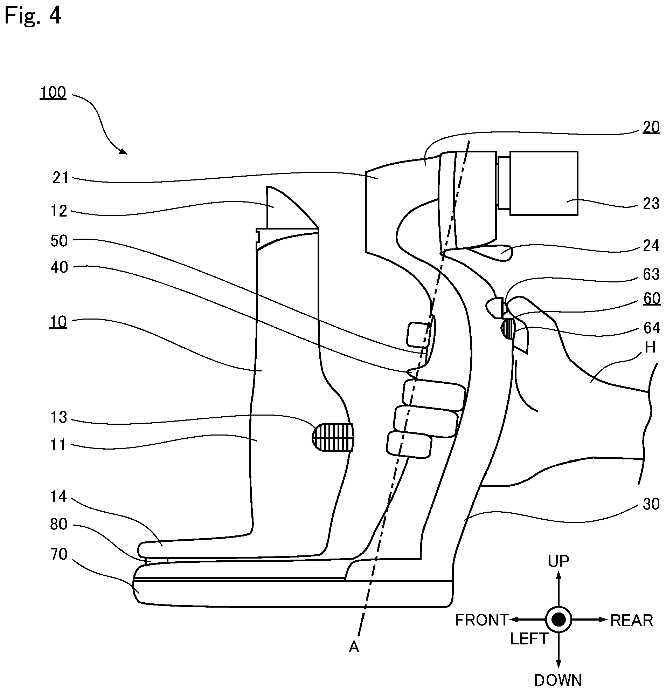

Fig. 4 is an external view when an operator holds the medical examination device 100 corresponding to at least one of the embodiments of the present invention with one hand. As illustrated in Fig. 4, the grip unit 30 is held so as to be sandwiched between the thumb and the index finger of the hand H of the operator. The grip unit 30 is divided into an index finger side (front side in Fig. 4) where four fingers are located and a thumb side (rear side in Fig. 4) where one thumb is located. Specifically, the middle finger, the ring finger, and the little finger are positioned on the index finger side in addition to the index finger. The finger hook unit 40 is provided on the index finger side and is hooked on the middle finger of the hand H. That is, in Fig. 4, the middle finger of the hand H is in a state of receiving the load of the finger hook unit 40. The index finger of the hand H is in contact with the index finger side operation unit 50 and is in an operable state. In addition, the thumb of the hand H is in contact with the thumb side operation unit 60 and is in an operable state. Note that a plane that passes through the one-dot chain line A and divides the thumb side and the index finger side into two is the above-described reference plane. The medical examination device 100 of the present example is a device in which the load balance is configured such that the center of gravity of the entire device is located on the index finger side when the thumb side and the index finger side are divided into two by the reference plane.

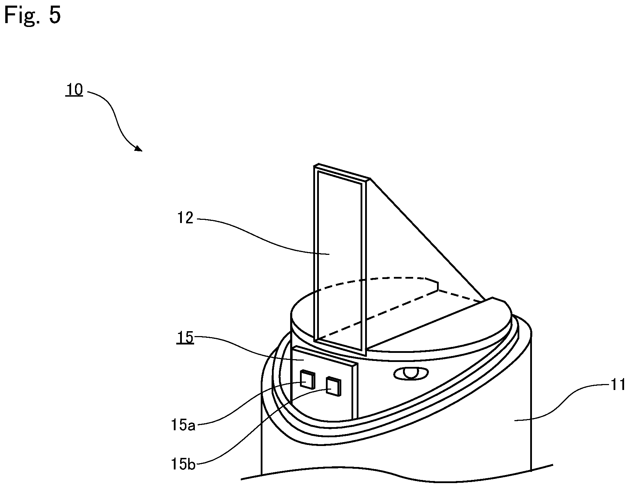

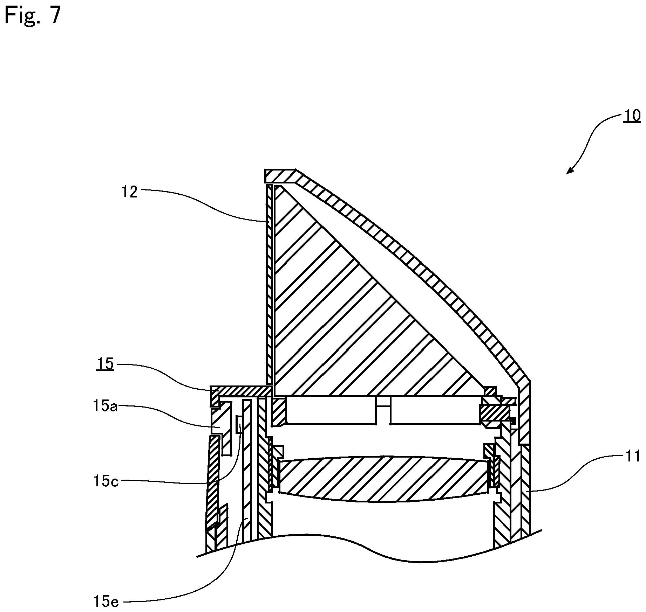

Meanwhile, as for the irradiation unit (illumination optical system) 10, the medical examination device 100 in the present example is characterized in that a separate light source installation unit 15 for installing a light source capable of performing irradiation control independently of the configuration for irradiation with slit light is provided, and in that functions of both irradiation with slit light and irradiation with a separate light source are realized in a compact configuration. Hereinafter, a detailed configuration of the irradiation unit 10 in the medical examination device 100 of the present example will be described with reference to Figs. 5 to 9. Note that, in the following description, a case where a light source for background illumination (first light) and a light source for fluorescence observation illumination (second light) are adopted as the separate light sources will be described as an example, but this is merely an example, and the number of separate light sources to be adopted and the application of the separate light source to be adopted can be variously set.

Figs. 5 to 9 are a perspective view and a cross-sectional view illustrating an example of a configuration of a portion of the irradiation unit 10 in the medical examination device 100 corresponding to at least one of the embodiments of the present invention.

As illustrated in Fig. 5, the irradiation unit 10 in the medical examination device 100 of the present example is provided with a background illumination irradiation port (first light irradiation port) 15a and a fluorescence observation illumination irradiation port (second light irradiation port) 15b which are a part of the configuration of the separate light source installation unit 15 at a portion different from the irradiation port 12 for irradiation with the slit light. Specifically, the background illumination irradiation port (first light irradiation port) 15a and the fluorescence observation illumination irradiation port (second light irradiation port) 15b are provided slightly below the irradiation port 12 provided at the distal end portion of the irradiation tube 11.

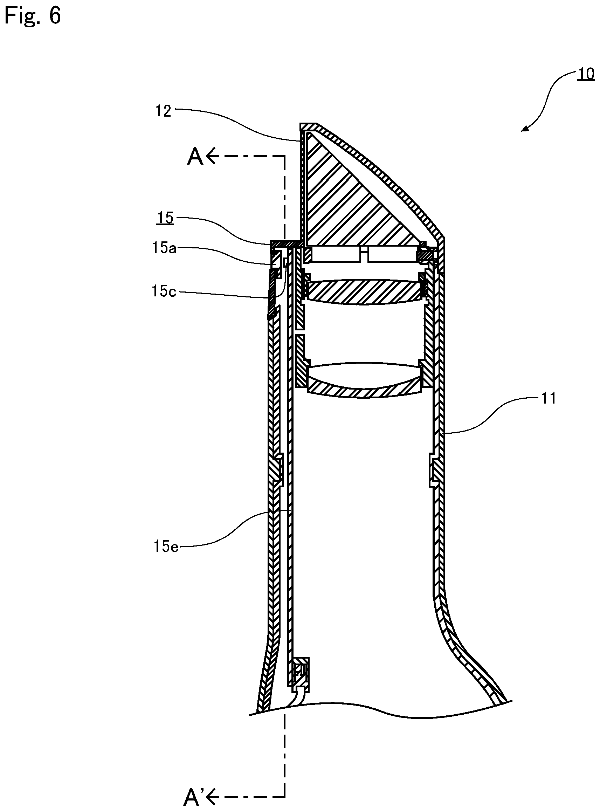

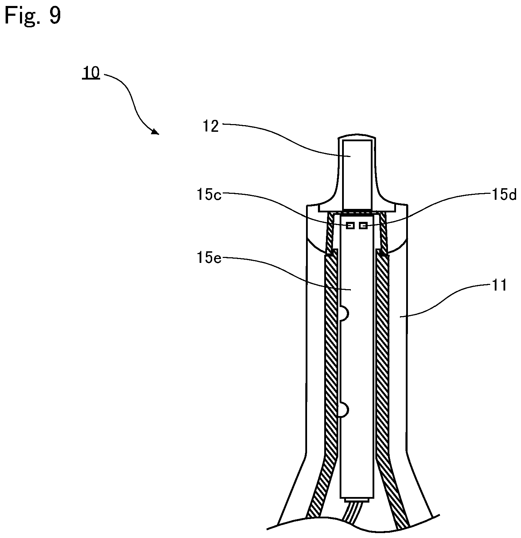

Furthermore, as illustrated in Figs. 6 to 8, a background illumination light source (first light source) 15c and a fluorescence observation illumination light source (second light source) 15d are installed inside each of the background illumination irradiation port (first light irradiation port) 15a and the fluorescence observation illumination irradiation port (second light irradiation port) 15b. A method of installing the background illumination light source (first light source) 15c and the fluorescence observation illumination light source (second light source) 15d is not particularly limited, but for example, it is conceivable to install them by a substrate 15e on which a white LED as the background illumination light source (first light source) 15c and a blue LED as the fluorescence observation illumination light source (second light source) 15d are mounted. By installing the substrate 15e along the inner wall of the irradiation tube 11, the arrangement of the background illumination light source (first light source) 15c and the fluorescence observation illumination light source (second light source) 15d is realized without disturbing the optical path of the slit light passing through the central portion of the irradiation tube 11 and emitted from the irradiation port 12. As illustrated in Fig. 9, the substrate 15e is disposed along the inner wall of the cylindrical irradiation tube 11 while securing the area necessary for the circuit configuration by extending the substrate 15e in the longitudinal direction of the irradiation tube 11. As illustrated in Fig. 9, by shortening the length of the short side of the substrate, the substrate can be disposed at a position close to the inner wall of the irradiation tube 11. Note that the substrate 15e may be extended in the longitudinal direction beyond the necessary area. This is because the substrate 15e needs to be disposed in a narrow place such as the inside of the irradiation tube 11. Therefore, if the substrate 15e is short and a wiring portion such as a harness is long, there is a risk that the harness may be pinched during assembly. Therefore, in a case where there is a space in the irradiation tube 11, if the substrate 15e is extended within the range, the length of the harness can be relatively shortened, so that the risk of pinching the harness at the time of assembly can be reduced, and workability can be improved.

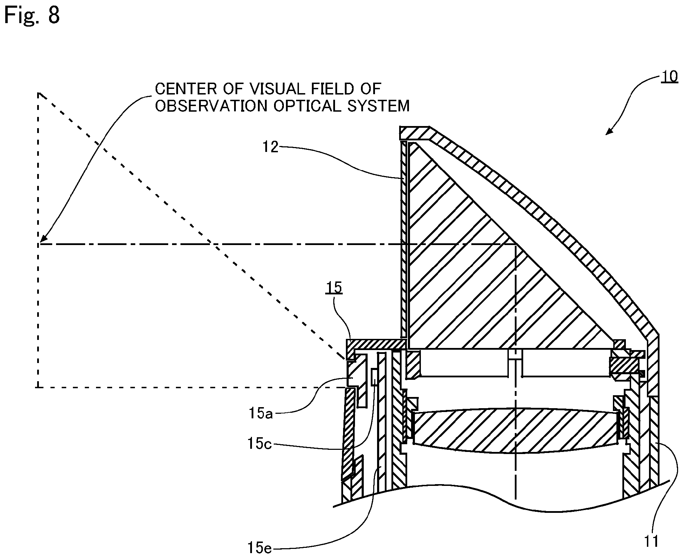

In addition, as illustrated in Fig. 8, it is preferable that the center of the visual field of the observation optical system and the center of the illumination area of the separate light source substantially coincide with each other. Here, the center of the visual field of the observation optical system means the center of the visual field when looking into the observation unit (observation optical system) 20. In addition, the irradiation direction of the slit light is also configured to be directed toward the center of the visual field of the observation optical system. Any configuration may be used to substantially match the center of the visual field of the observation optical system with the center of the illumination area of the separate light source, but for example, it is conceivable to set the shapes and sizes of the opening of the background illumination irradiation port (first light irradiation port) 15a and the fluorescence observation illumination irradiation port (second light irradiation port) 15b so as to be an opening that limits the illumination area of the background illumination light source (first light source) 15c and/or the fluorescence observation illumination light source (second light source) 15d. In addition, by adopting a diffusion plate for the background illumination irradiation port (first light irradiation port) 15a and the fluorescence observation illumination irradiation port (second light irradiation port) 15b to form a first diffusion plate and a second diffusion plate, it is possible to emit the light of the first light source and the second light source while diffusing the light.

For the background illumination light source (first light source) 15c and/or the fluorescence observation illumination light source (second light source) 15d in the separate light source installation unit 15 described with reference to Figs. 5 to 9, operation buttons are assigned to be different from the index finger side operation unit 50, to which the irradiation with the slit light is assigned, so as to be controlled independently of the irradiation with the slit light. For example, it is conceivable to assign the operation button 61 as a button for switching the light emission of the background illumination light source (first light source) 15c and assign the operation button 63 as a button for switching the light emission of the fluorescence observation illumination light source (second light source) 15d. In this way, different operation buttons are assigned to each, and then a control unit (not illustrated) for independently controlling the irradiation with the slit light, the background illumination light, and the fluorescence observation illumination light is provided. This provides a configuration in which irradiation control can be performed independently regardless of the state of other irradiation lights.

By assigning an operation button to each of the irradiation with the slit light, the background illumination light (hereinafter, it is also referred to as "background light"), and the fluorescence observation illumination light (hereinafter, it is also referred to as "fluorescence observation light") so that they can be controlled independently, irradiation control can be independently performed regardless of the state of other irradiation light. However, if the irradiation with the slit light, the background light, and the fluorescence observation light can be performed without any limitation, a plurality of irradiation lights are simultaneously emitted to the examinee's eye, which may cause discomfort and stress to the subject. Therefore, as an example, it is conceivable to perform control to prohibit an operation for irradiation with one of the combinations of specific illumination lights during irradiation with the other. Furthermore, as another example, it is conceivable to perform exclusive irradiation control of interrupting irradiation with one and starting irradiation with the other in a case where an operation for irradiation with one is performed during irradiation with the other for a specific combination of illumination lights.

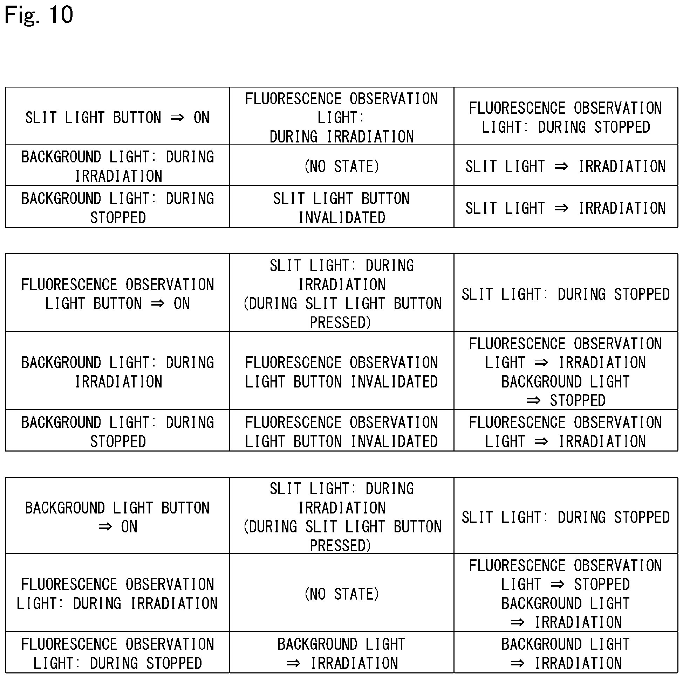

Fig. 10 is an explanatory diagram illustrating an example of a relationship between an irradiation state of each irradiation light and availability of button operation in the medical examination device 100 corresponding to at least one of the embodiments of the present invention. First, a case where a button for emitting slit light is operated will be described. When the slit light is turned on, in a case where the background light is turned off and the fluorescence observation light is turned off, and in a case where the background light is turned on and the fluorescence observation light is turned off, the irradiation with the slit light is permitted. However, in a case where the background light is turned off and the fluorescence observation light is turned on when the slit light is turned on, the irradiation with the slit light is prohibited, that is, the operation of the irradiation button of the slit light is invalidated. Note that, in the present example, since the background light and the fluorescence observation light are exclusively controlled, a state where the background light is turned on and the fluorescence observation light is turned on cannot occur.

Next, a case where a button for emitting the fluorescence observation light is operated will be described. In a case where the slit light is turned off and the background light is turned off when the fluorescence observation light is turned on, the irradiation with the fluorescence observation light is permitted. In a case where the slit light is turned off and the background light is turned off when the fluorescence observation light is turned on, the irradiation with the fluorescence observation light is permitted. In a case where the slit light is turned off and the background light is turned on when the fluorescence observation light is turned on, the irradiation with the fluorescence observation light is permitted and the background light is stopped (for exclusive control). In a case where the slit light is turned on when the fluorescence observation light is turned on, the irradiation with the fluorescence observation light is prohibited regardless of on/off of the background light. That is, the operation on the irradiation button for the fluorescence observation light is invalidated.

Next, a case where a button for emitting background light is operated will be described. In a case where the slit light is turned off and the fluorescence observation light is turned off when the background light is turned on, the irradiation with the background light is permitted. In a case where the slit light is turned off and the fluorescence observation light is turned on when the background light is turned on, the irradiation with the background light is permitted and the fluorescence observation light is stopped (for exclusive control). In a case where the slit light is turned on and the fluorescence observation light is turned off when the background light is turned on, the irradiation with the background light is permitted. Note that, in the present example, since control is performed to prohibit an operation for irradiation with one of the slit light and the fluorescence observation light during irradiation with the other, a state where the slit light is turned on and the fluorescence observation light is turned on cannot occur.

By performing such control, it is possible to prevent adverse effects and stress on the subject due to simultaneous irradiation. For example, in a state where a user holds the grip unit 30 of the medical examination device 100 of the present example, an erroneous operation of the slit light button (the index finger side operation unit 50) is likely to occur, and thus, when the fluorescence observation light is turned on, performing control to prohibit the operation of the slit light is effective in preventing malfunction. In addition, since a background light button (the operation button 61) and a fluorescence observation light button (the operation button 63) have a relationship in which there is a relatively small number of erroneous operations and even if an erroneous operation is performed, there is no big problem, each of the background light button and the fluorescence observation light button are controlled so as to be able to be turned on/off at any time instead of controlling the background light button and the fluorescence observation light button exclusively, whereby the operations of the background light button and the fluorescence observation light button are effective regardless of the irradiation states of the background light and the fluorescence observation light. As a result, the background light button and the fluorescence observation light button can be operated regardless of the irradiation state of the background light and the fluorescence observation light, and a bothersome operation such as turning off one and then turning on the other becomes unnecessary, so that an effect of improving convenience for a user can be obtained.

As described above, the medical examination device according to the present invention includes: an observation optical system that observes a subject; an illumination optical system having a slit light generation unit that generates slit light, a first light generation unit that generates a first light different from the slit light, and an exterior in which a slit light irradiation port that emits the slit light and a first light irradiation port that emits the first light are formed at different positions, and a control unit that controls the illumination optical system. Since the first light generation unit is arranged to have a first light source arranged at a position where the first light can be emitted from the first light irradiation port, it is possible to realize a configuration in which the slit light irradiation port and the first light irradiation port are formed at different positions and can be independently controlled.

In addition, the illumination optical system includes an irradiation tube that allows the slit light emitted from the slit light generation unit to pass therethrough, reflects the slit light at an end portion, and irradiates the subject with the slit light, and a substrate on which the first light source is mounted is disposed as the first light generation unit between a passing region of the slit light inside the irradiation tube and an outer wall (exterior) of the irradiation tube so as not to enter the passing region of the slit light. Therefore, even if the first light generation unit that can be controlled independently of the irradiation with the slit light is provided, it is possible to prevent the size of the medical examination device from increasing and maintain the size.

In addition, since the substrate is arranged so as to secure an area necessary for the circuit configuration by extending in the longitudinal direction of the irradiation tube, the first light generation unit can be installed by effectively using a limited space in the irradiation tube.

In addition, since the control unit independently controls the irradiation with the slit light and the irradiation with the first light, it is possible to realize an operation that cannot be conventionally performed, for example, simultaneous irradiation with the slit light and the background light.

In addition, since the first light generation unit includes a first diffusion plate and is arranged so as to emit the first light from the first light irradiation port via the first diffusion plate, it is possible to diffuse the first light by the first diffusion plate and emit the first light. Therefore, it is possible to adopt a light source having strong directivity, for example, an LED as the first light source, and as a result, it is possible to realize downsizing of the configuration of a separate light source installation unit.

In addition, since the first light irradiation port is arranged so as to have an opening that limits the illumination area of the first light so that the center of the visual field of the observation optical system substantially coincides with the center of the illumination area of the first light, it is possible to prevent discomfort caused by deviation of the center of the visual field of the observation optical system from the center of the illumination area of the first light.

In addition, the illumination optical system includes a second light generation unit that generates a second light different from the slit light and the first light, and the exterior of the illumination optical system is arranged so as to have a second light irradiation port that emits the second light in the vicinity of the first light irradiation port. Therefore, it is possible to select and emit the first light or the second light as the light different from the slit light.

In addition, a grip unit that can be held by the operator with one hand is provided, the grip unit includes a slit light button for instructing start/stop of irradiation with slit light, a first light button for instructing start/stop of irradiation with first light, and a second light button for instructing start/stop of irradiation with second light, and the slit light button, the first light button, and the second light button are arranged so as to be operable by the operator's holding hand in a state where the operator holds the grip unit, and thus, it is possible to perform an operation regarding irradiation with the slit light, the first light, and the second light with the hand holding the grip unit.

In addition, since the slit light button and the second light button are arranged so as to be operable with different fingers and the first light button and the second light button are arranged so as to be operable with the same finger in a state where the operator holds the grip unit, it is possible to further reduce the possibility of erroneous operation by assigning different fingers to the operation for the combination of the slit light button and the second light button. Of course, in a case where the slit light button and the first light button are arranged so as to be operable by different fingers, the possibility of erroneous operation by these buttons can also be reduced.

In addition, since the second light generation unit includes the second diffusion plate and is arranged so as to emit the second light from the second light irradiation port via the second diffusion plate, it is possible to diffuse the second light by the second diffusion plate and emit the second light. Therefore, it is possible to adopt a light source having strong directivity, for example, an LED as the second light source, and as a result, it is possible to realize downsizing of the configuration of the separate light source installation unit.

In addition, since the control unit is set to perform control so as to prohibit the irradiation with the second light during the irradiation with the slit light and to prohibit the irradiation with the slit light during the irradiation with the second light, as for the slit light and the second light for which switching of the irradiation light by an erroneous operation is desired to be prevented, control is performed such that the irradiation with one of the slit light and the second light cannot be started unless the irradiation with the other is stopped, and thus, it is possible to prevent the switching of the irradiation light by the erroneous operation.

In addition, since the control unit is set to perform control so as to exclusively emit the first light and the second light, the first light and the second light are exclusively controlled, so that the irradiation light can be easily switched while preventing simultaneous irradiation.

In addition, since the second light irradiation port is arranged so as to have an opening that limits the illumination area of the second light so that the center of the visual field of the observation optical system substantially coincides with the center of the illumination area of the second light, it is possible to prevent discomfort caused by deviation of the center of the visual field of the observation optical system from the center of the illumination area of the second light.

### Reference Signs List

100 medical examination device
10 irradiation unit
11 irradiation tube
12 irradiation port
13 disk operation unit
14 swing unit
15 separate light source installation unit
15a background illumination irradiation port (first light irradiation port)
15b fluorescence observation illumination irradiation port (second light irradiation port)
15c background illumination light source (first light source)
15d fluorescence observation illumination light source (second light source)
15e substrate
20 observation unit
21 observation casing
22 right-eye observation unit
23 left-eye observation unit
24 zoom lever
30 grip unit
40 finger hook unit
50 index finger side operation unit
60 thumb side operation unit
61 to 63 operation button
64 operation dial
70 base unit
80 irradiation angle adjustment unit
90 scale unit

## Claims

1. A medical examination device comprising:
an observation optical system that observes a subject;
an illumination optical system including a slit light generation unit that generates slit light, a first light generation unit that generates first light different from the slit light, and an exterior in which a slit light irradiation port that emits the slit light and a first light irradiation port that emits the first light are formed at different positions; and
a control unit that controls the illumination optical system,
wherein the first light generation unit includes a first light source disposed at a position where the first light capable of being emitted from the first light irradiation port.

2. The medical examination device according to claim 1,
wherein the illumination optical system includes an irradiation tube that allows the slit light emitted from the slit light generation unit to pass therethrough, reflects the slit light at an end portion, and irradiates the subject with the slit light, and
a substrate on which the first light source is mounted is disposed as the first light generation unit between a passing region of the slit light inside the irradiation tube and an outer wall (exterior) of the irradiation tube so as not to enter the passing region of the slit light.

3. The medical examination device according to claim 2,
wherein the substrate is extended in a longitudinal direction of the irradiation tube to secure an area necessary for a circuit configuration.

4. The medical examination device according to any one of claims 1 to 3,
wherein the control unit independently controls irradiation with the slit light and irradiation with the first light.

5. The medical examination device according to any one of claims 1 to 4,
wherein the first light generation unit includes a first diffusion plate and emits the first light from the first light irradiation port via the first diffusion plate.

6. The medical examination device according to any one of claims 1 to 5,
wherein the first light irradiation port has an opening that limits an illumination area of the first light such that a center of a visual field of the observation optical system and a center of the illumination area of the first light substantially coincide with each other.

7. The medical examination device according to any one of claims 1 to 6,
wherein the illumination optical system includes a second light generation unit that generates a second light different from the slit light and the first light, and
the exterior of the illumination optical system has a second light irradiation port that emits the second light in the vicinity of the first light irradiation port.

8. The medical examination device according to claim 7, comprising
a grip unit that capable of being held by a user with one hand,
wherein the grip unit includes:
a slit light button configured to instruct start/stop of irradiation with the slit light;
a first light button configured to instruct start/stop of irradiation with the first light; and
a second light button configured to instruct start/stop of irradiation with the second light, and
the slit light button, the first light button, and the second light button are arranged to be operable by the user's holding hand in a state where the user holds the grip unit.

9. The medical examination device according to claim 8,
wherein in a state where the user holds the grip unit,
the slit light button and the second light button are arranged to be operable by different fingers, and
the first light button and the second light button are arranged so as to be operable by the same finger.

10. The medical examination device according to any one of claims 7 to 9,
wherein the second light generation unit includes a second diffusion plate and emits the second light from the second light irradiation port via the second diffusion plate.

11. The medical examination device according to any one of claims 7 to 10,
wherein the control unit controls to prohibit the irradiation with the second light during the irradiation with the slit light and to prohibit the irradiation with the slit light during the irradiation with the second light.

12. The medical examination device according to any one of claims 7 to 11,
wherein the control unit controls to exclusively emit the first light and the second light.

13. The medical examination device according to any one of claims 7 to 12,
wherein the second light irradiation port has an opening that limits an illumination area of the second light such that a center of a visual field of the observation optical system and a center of the illumination area of the second light substantially coincide with each other.
